# EUROPEAN PATENT APPLICATION

(11) **EP 2 848 222 A2**
(43) Date of publication of application: **18.03.2015**
(21) Application number: 14183011.7
(22) Date of filing: 01.09.2014
(51) Int. Cl.: A61B 17/70

(54) **Vertebral fixation apparatus**

(30) Priority: 14.09.2013 TW 102133297
(71) Applicant: Paonan Biotech Co., Ltd, Taipei City 114 (TW)
(72) Inventor: Yeh, Chung-Chun, 114 Taipei City (TW)
(74) Representative: Schwerbrock, Florian

(57) **Abstract**

A vertebral fixation apparatus includes a fixation plate (12) respectively secured to the root portions of adjacent transverse processes (T, T'), rather than the adjacent spinous processes, a superior saddle portion (111) and an inferior saddle portion (112) respectively formed on an upper and a lower portion of the fixation plate for holding a superior spinous process (S1) and an inferior spinous process (S2) within the superior and inferior saddle portions for stably securing the fixation plate on the adjacent vertebrae for distracting the adjacent vertebrae for relieving the pressure and pain of the spinal cord and nerve.

## Description

### Background of the Invention:

In order for treating spinal conditions such as stenosis, a fixation device is provided for fixing the adjacent spinous processes of the two neighboring vertebrae to decompress the spinal cord and nerve so as to eliminate the pressure on the spinal vessel or nerve for relieving the back pain or other spinal symptoms.

US Patent 8388657, 8475497, 8591548 or 8603142 disclosed a spinous process fixation apparatus. US Patent 8,591,548 disclosed a spinous process fusion plate assembly including two fixation plates (10, 20) to "clamp" both sides of the adjacent spinous processes, having projections (15) inserted into the spinous processes when fastened by a locking element (40) provided on the brace (30) for linking the two plates (10, 20) together.

Such a prior art may have the following drawbacks:
1. The projections 15 are stuck into the spinous processes to easily cause breakage or fracture of the spinous processes, especially when "clamping" the two plates (10, 20) under pressure on the weak spinous processes, easily causing fixation failure.
2.The two plates (10, 20) are fastened on the weak spinous processes, not firmly fixed to the strong vertebral portions (such as on the stronger root portion of transverse process), and are easily loosened, when bending or twisting the patient's body, to be separated from the vertebrae to thereby lose its effect for relieving the pressure on pinched nerves.
3.The plates (10, 20) are generally perpendicular to the vertebral surface, unable to cover the surgery area such as when removing partial vertebral arch or lamina, thereby being unable to preclude the tissue re-growth into the surgery area and thereby still pressurizing the spinal nerve to cause pain.

The present inventor has found the drawbacks of the prior art, and invented the present fixation apparition for stably holding the adjacent spinous processes.

### Summary of the Invention:

The object of the present invention is to provide a vertebral fixation apparatus including a fixation plate respectively secured to the root portions of adjacent transverse processes, rather than the adjacent spinous processes, a superior saddle portion and an inferior saddle portion respectively formed on an upper and a lower portion of the fixation plate for holding the superior spinous process and the inferior spinous process within the superior and inferior saddle portions for stably securing the fixation plate on the adjacent vertebrae for distracting the adjacent vertebrae for relieving the pressure and pain of the spinal cord and nerve.

Another object of the present invention is to provide a fixation apparatus which may cover the surgery area of the vertebral arch to preclude the tissue re-growth into the surgery area to prevent from pressurizing on the pinched nerves, thereby relieving the pain.

### Brief Description of the Drawings:

Fig. 1 is an exploded view of the present invention.
Fig. 2 is a partial enlarged view for the micro-lattice structure of the present invention.
Fig. 3 is a perspective view of the present invention when holding adjacent spinous processes.
Fig. 4 is a top view illustration based on Fig. 3 of the present invention.
Fig. 5 shows another preferred embodiment of the present invention.
Fig. 6 shows still another preferred embodiment of the present invention.
Fig. 7 is a top view illustration based on Fig. 6.
Fig. 8 shows further preferred embodiment of the present invention.
Fig. 9 shows still further preferred embodiment of the present invention.
Fig. 10 shows a single-leaf plate member combined with a holding means of the present invention.
Fig. 11 shows an embodiment of the present invention when combining the elements of Fig. 10.

### Detailed Description:

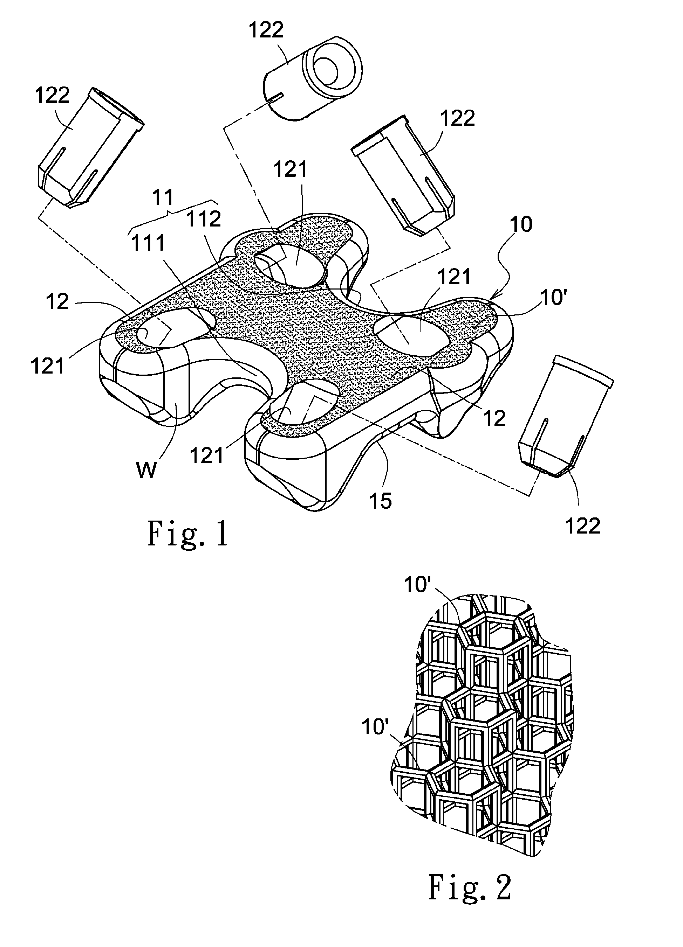

As shown in Figs. 1∼4, the present invention discloses a vertebral fixation apparatus 10 for holding two spinous processes S1, S2 of the two adjacent vertebrae S, S' in order for stably spanning the two adjacent vertebrae S, S' with a proper intervertebral distance, thereby preventing compressing on the spinal blood vessel or nerve and eliminating the back pain or spinal symptoms.

The vertebral fixation apparatus 10 of the present invention comprises: a fixation plate 12 having a first saddle portion 111 formed on a first (or superior) portion of the fixation plate 12 for holding a first (or superior) spinous process S1, and a second saddle portion 112 formed on a second (or inferior) portion of the fixation plate 12, opposite to the first saddle portion 111, for holding a second (or inferior) spinous process S2; a first pair of screws 13 respectively locking the first saddle portion 111 into root portions of a pair of first (or superior) transverse processes T; and a second pair of screws 13 respectively locking the second saddle portion 112 into root portions of a pair of second (or inferior) transverse processes T'.

The first saddle portion 111 and the second saddle portion 112 may be cooperatively defined as "a holding means" 11 for stably holding the adjacent spinous processes S1, S2 on the fixation plate 12.

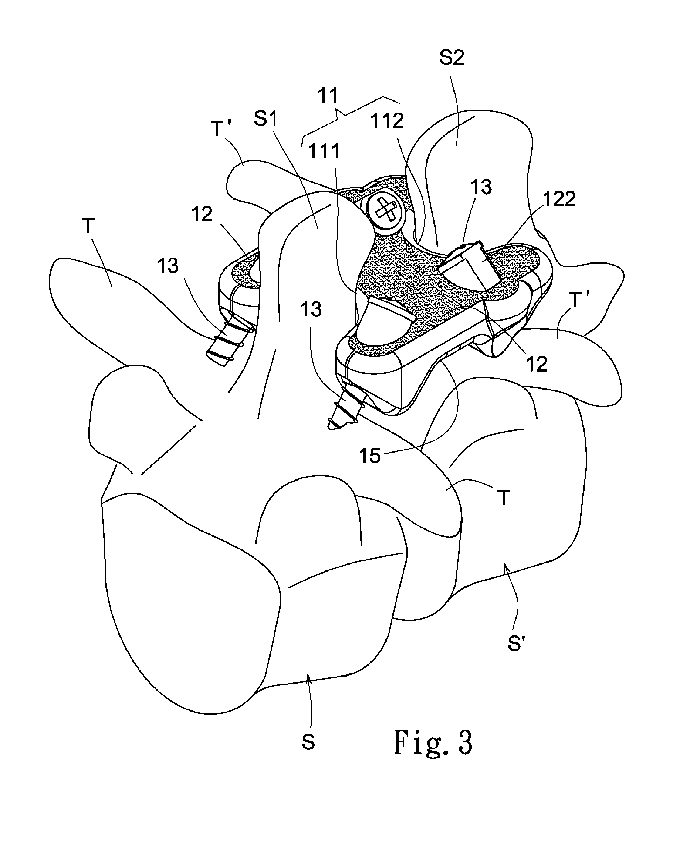

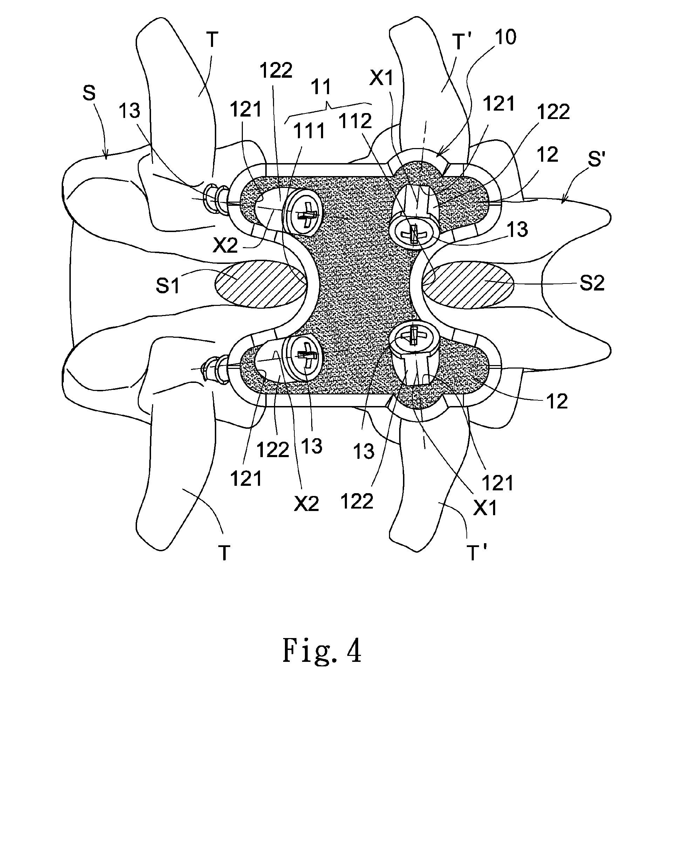

The fixation plate 12 is locked into the root portions of the transverse processes T, T' by the four screws 13 as shown in Figs. 4 and 3. Since the transverse process is the strongest part of the vertebra, the fixation apparatus 10 of the present invention will be strongly and stably secured on the adjacent vertebrae S, S'. The fixation apparatus 10 is not fixed on the spinous processes S1, S2, which are weak to be vulnerably broken in comparison with the stronger transverse processes T, T', so that the breakage of spinous processes S1, S2 can be prevented in accordance with the present invention.

The fixation apparatus 10, once secured on the adjacent vertebrae S, S', will decompress the spinal cord and nerve in the vertebrae to prevent or eliminate the back pain or spinal symptoms. Meanwhile, such a fixation apparatus 10 may serve as a barrier to protect or shield the vertebra S', which may be subjected to surgery for treating spinal stenosis (such as by removing or cutting lamina or vertebral arch), in order to prevent intrusion of newly growing tissue into the surgery vertebral area which may re-compress the spinal nerve to cause back pain or other uncomfortable symptoms.

Each screw 13 is locked into the root portion of the transverse process T, T' by inserting through a fixing hole 121 formed in (or adjacent to) the saddle portion 111, 112 on the fixation plate 12.

Each fixing hole 121 may be pre-inserted with a screw sleeve 122 therein, and then each screw 13 is locked into the fixing hole 121 by passing through each screw sleeve 122. The shape of each screw 122 and fixing hole 121 may not be a circular or cylindrical shape in order to prevent from its self-rotation, thereby enhancing the locking stability of each screw 13 and the fixing plate 12 of the present invention.

Each fixing hole 121 may be obliquely formed in the fixation plate 10, not perpendicular to the surface of the transverse process T, T' as shown in Figs. 4 and 3.

An inferior fixing hole 121 adjacent the inferior spinous process S2 may be obliquely formed in a root portion of the inferior transverse process T' along a first axis X1 which is generally projectively aligned with or parallel to an orientation of the inferior transverse process T' as shown in Fig. 4; while a superior fixing hole 121 adjacent to the superior spinous process T may be obliquely formed in a root portion of the superior transverse process T along a second axis X2 which is generally projectively perpendicular to the first axis X1; whereby upon fixing of each (lower) screw 13 into each inferior fixing hole 121 along the first axis X1, the two (lower) screws 13 will be secured into the two inferior fixing holes 121 to firmly secure the pair of inferior transverse processes T', the inferior spinous process S2 with the fixation plate 12 latitudinally; and upon fixing of each (upper) screw 13 into each superior fixing hole 121 along the second axis X2, the two (upper) screws 13 will be secured into the two superior fixing holes to firmly secure the superior spinous process S, the superior transverse processes T, with the fixation plate 12 longitudinally, thereby fixing the fixation plate 12 stably firmly among the superior spinous process S1, the superior transverse processes T, the inferior spinous process S2 and the inferior transverse processes T'.

Since the superior spinous process S1 and the inferior spinous process S2 are snugly held in the superior saddle portion 111 and the inferior saddle portion 112, without being squeezed, compressed, clamped or "invasively" stuck, the spinous processes S1, S2 will not be broken or fractured and will be stably held by the fixation plate 12 for protecting the vertebrae safely.

The definition of the axis X1 or X2 as aforementioned is just provided for explanation purpose in the present invention. The orientations and fixing methods for fixing the screws 13 into the vertebral portion are, however, not limited in this invention.

The saddle portion 111 or 112 is formed like a "horse saddle" having a depression or recess as recessed in two elevations or peaks for holding each spinous process S1 or S2 within the recess. In other words, each spinous process S1 or S2 is "riding" on each saddle portion 111 or 112, and the two spinous processes S1, S2 are thus stably held in between the two saddle portions 111, 112 especially as shown in Figs. 4 and 3. As any two adjacent vertebrae S, S' are spanned or spread apart by the fixation plate 12 and the adjacent vertebrae are also firmly stably "linked" by the fixation apparatus 10 of the present invention, the vertebrae will then be decompressed, distracted, or spaced to release the pressure on the spinal cord or nerve to relieve the patient's back pain accordingly.

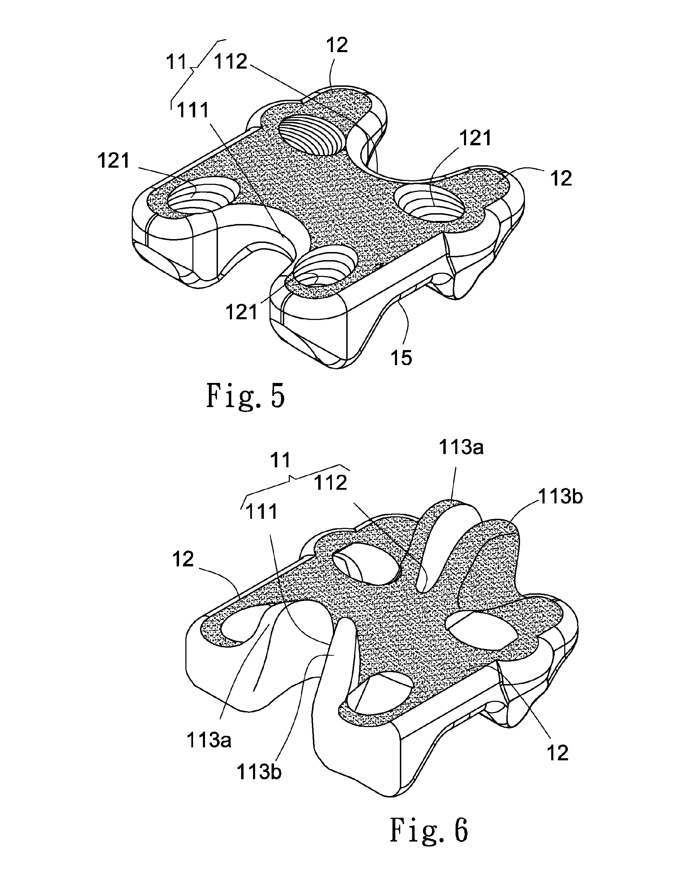

As shown in Fig. 5, each fixing hole 151 is formed with female threads 121a to be engaged with the male threads as formed on each screw 13. Also, the screw 13 may be a self-attack screw which may be locked into the vertebral portion in situ during the locking process of the screw. However, it should be carefully considered by evaluating the patient's bone density or strength whenever applying the self-attack screw which may be more destructive than the other screws.

As shown in Figs. 1∼3, the fixation apparatus 10 of the present invention may be formed as micro-lattice hollow structure 10', each micro lattice being shaped as: triangular, hexagonal, beehive, polygonal, regular or irregular porosity shapes or structures, adapted for fusion of tissue or bone re-growth into the micro-lattice hollow structure 10' to thereby enhance the vertebral or spinal strength. A well fusion on the fixation apparatus 10 and the linked vertebrae will diffuse any external force to be homogeneously acting on the vertebrae, not merely concentrating the external force on the fixing screws 13 to easily cause fixing failure of the fixation apparatus.

The size of each micro lattice may range 100 microns through 400 microns for an optimum fusion effect, but not limited in this invention. For strengthening the whole structure, a solid metallic frame or protection wall W may be provided for fencing the micro-lattice structure.

The fixation apparatus 10 of the present invention may be formed by 3-D printing and shaping technique. The fixation apparatus 10 may be made of: titanium, stainless steel, PEEK, metal or alloy materials. Both flexibility and rigidity of the fixation apparatus must be considered. The fixation apparatus 10 may also be formed by conventional mechanical processing or metallic injection molding process, not limited in this invention.

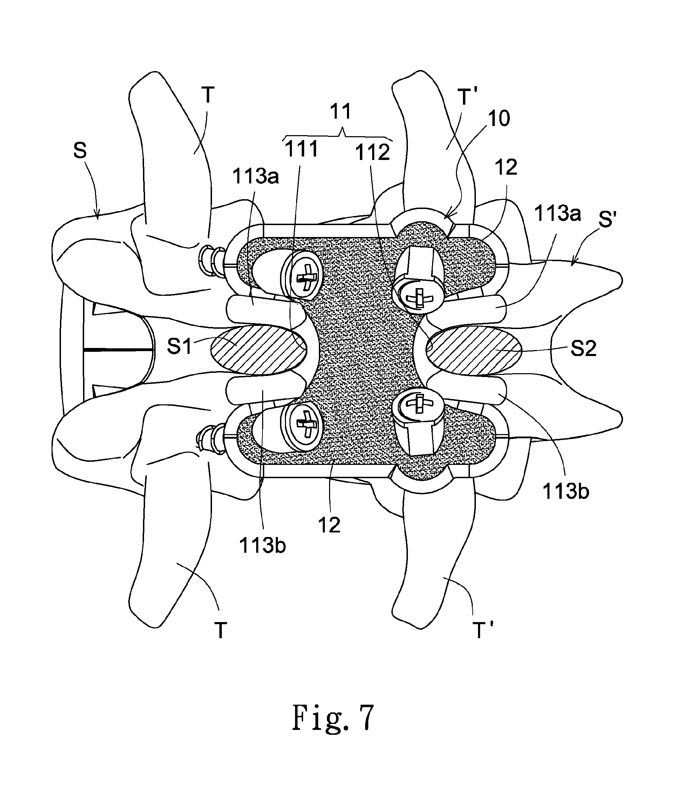

As shown in Figs. 6 & 7, each saddle portion 111 or 112 may be further protected with a U-shape or V-shape packing or protecting member 113a, 113b along a periphery of said saddle portion for well protection of each spinous process S1 or S2.

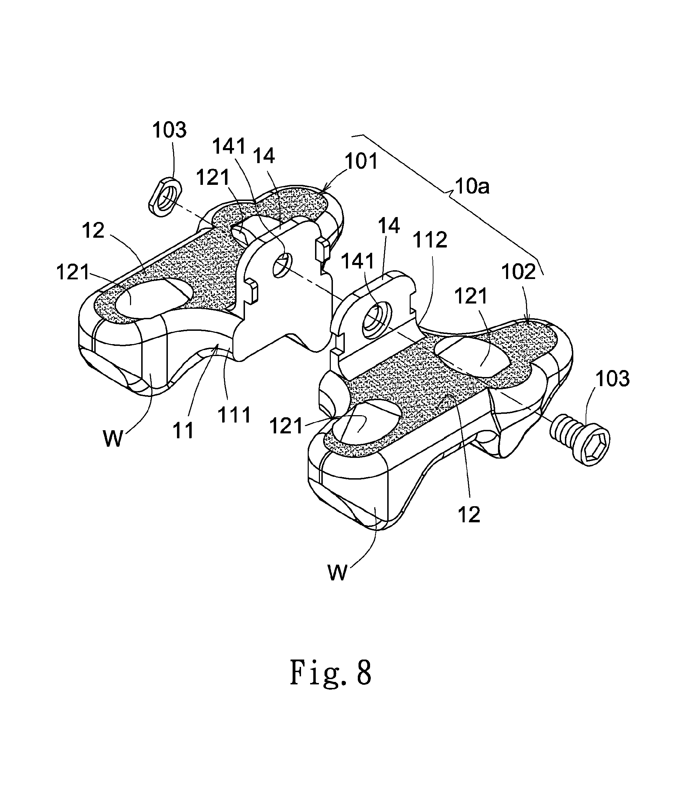

As shown in Fig. 8, the fixation apparatus 10a may be divided into two half plate members, namely, a left plate member 101 and a right plate member 102, each having a connecting projection 14, so that the two half plate members 101, 102 may be locked and combined by a fastening screw 103 which is provided to pass each screw opening 141 formed through each connecting projection 14.

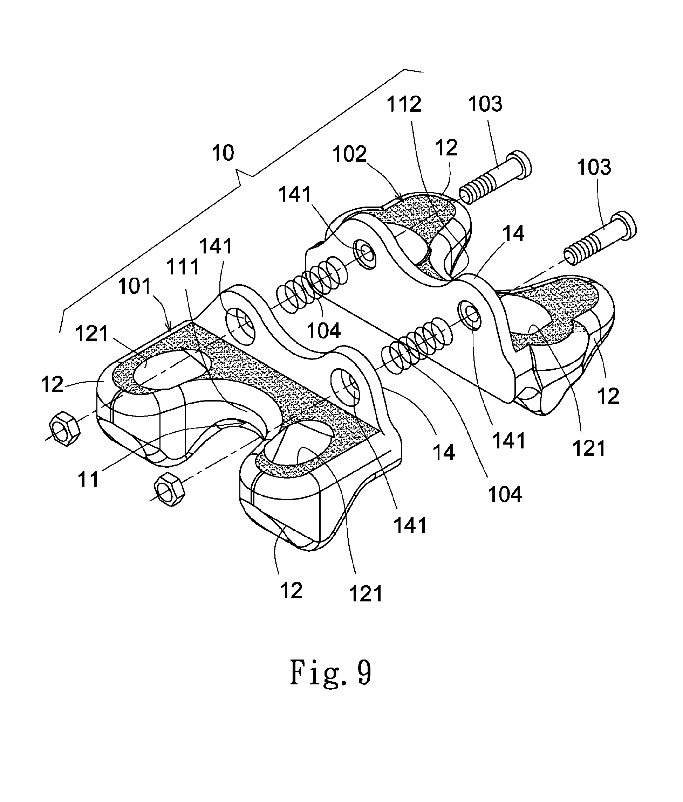

As shown in Fig. 9, the fixation apparatus 10b may be divided into a superior half plate member 101 and an inferior half plate member 102, which are combined by locking at least a (or two) fastening screw(s) 103 passing through screw opening(s) 141 formed through each connecting projection 14. A spring 104 may be jacketed on each fastening screw 103.

The two half plate members 101, 102 as shown in Figs. 9 and 8 may be further provided with a spacer (not shown) in between the two plate members 101, 102 for adjusting the length or width of the fixation apparatus to match the distance between two adjacent spinous processes S1, S2 or to match the shape or circumference of the spinous processes.

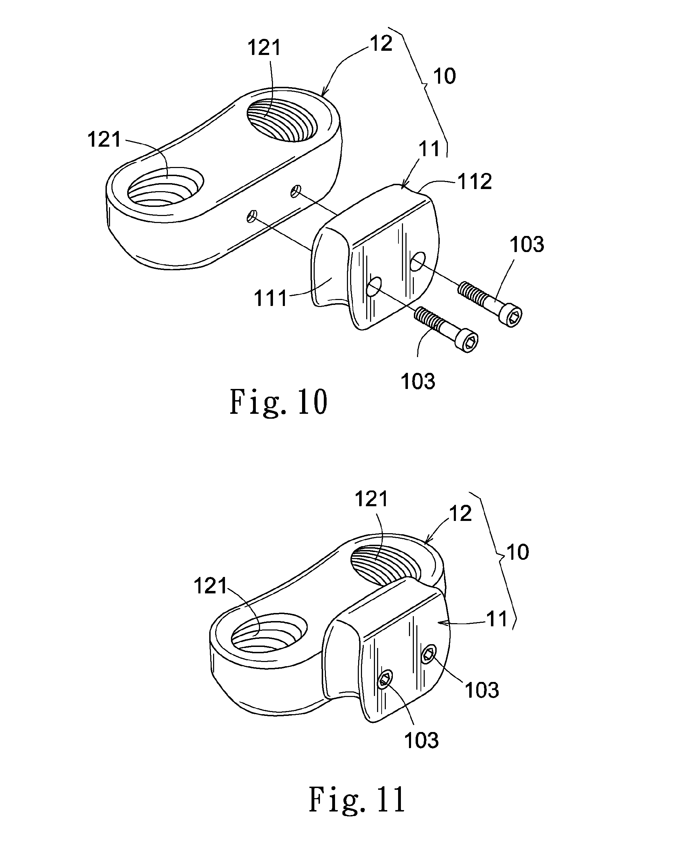

As shown in Figs. 10 and 11, the fixation apparatus 10 may be modified to be a single-leaf plate member 12 and a holding means 11 including a superior saddle portion 111 and an inferior saddle portion 112, having at least a (or two) fastening screw(s) 103 for locking the single-leaf plate member 12 with the holding means 11. Such a single-leaf plate member 12 may be a left plate member or a right plate member, depending upon the patient's requirement.

By the way, the minimally invasive surgery may be applied to a patient whose surgery area may be limited to be as minimum as possible, for example, just conducting invasive operation for a left vertebral portion, or for a right portion only, without requiring a large surgical area which may cause patient's uncomfortableness, pain, or even infection. The holding means 11 may be made with plural sizes for optional choices.

The fixation apparatus 10 of the present invention may be made with a plurality of sizes, for example, plural distances between the two saddle portions 111, 112 which may be provided for patient's selections.

A recess 15 is formed in a bottom of the fixation apparatus 10 as shown in Figs. 1 and 3 to form a "space" between the fixation plate 10 and the vertebral arch, thereby preventing compression on the spinal nerve and the back pain caused by the fixation apparatus.

In surgical operation, the vertebral arch may be partially removed to provide "space" for accommodating the spinal cord or nerve to relieve the pressure acting upon the spinal nerve. After the surgery operation, the fixation plate 10 of the present invention may be provided to cover the surgery area (the area by removing the partial vertebral arch) in order to preclude the intrustion of re-growing tissue to further compress the spinal nerve. The bone tissue will grow directly on the fixation plate 10 especially when formed as micro-lattice structure as shown in Figs. 1, 2 for well fusing the plate 10 and the neighboring vertebral portions. Some bone powders or bone cement may be filled into the proper areas of the fixation apparatus 10 and the vertebrae as held on the apparatus 10 for better fusion.

The two spinous processes S1, S2 of the adjacent vertebrae S, S' are "saddled" by the two saddle portions 111, 112 on the fixation plate 12 of the present invention, like riding on a house saddle, will be stably held by the fixation apparatus 10. No pressurized clamping, fastening or squeezing are acting on the spinous processes. No "invasive" sticking, intrusion, or piercing is performed on the weak spinous process. So, the spinous processes of the adjacent vertebrae can be well protected from breakage or fracture by the present invention.

The fixation apparatus 10 is provided to "link" the two adjacent vertebrae by locking the screws 13 onto the transverse processes T, T' which are relatively strong. So, the vertebrae may be stably fixed ready for a proper fusion.

By the aid of the oblique fixing holes for fixing screws 13 along axis X1 or X2, the fixation plate of the present invention will be firmly secured with the adjacent verhebrae latitudinally (X1, X1) and longitudinally (X2, X2) (Fig. 4), thereby enhancing a firm, stable, and reliable fixation of the vertebrae on the fixation plate for well protecting the spinal cord and nerve.

## Claims

1. A vertebral fixation apparatus comprising:
a fixation plate;
a superior saddle portion formed on an upper portion of said fixation plate, adapted for holding a superior spinous process in said superior saddle portion, said superior saddle portion adapted to be secured to at least a root portion of a superior transverse process;
an inferior saddle portion formed on a lower portion of said fixation plate, adapted for holding an inferior spionous process in said inferior saddle portion, said inferior saddle portion adapted to be secured to at least a root portion of an inferior transverse process; and
said fixation plate adapted to hold adjacent spinous processes in between said superior saddle portion and said inferior saddle portion, and said fixation plate adapted for covering at least a superior vertebral arch and an inferior vertebral arch.

2. A vertebral fixation apparatus according to Claim 1, wherein said fixation plate is formed as a micro-lattice hollow structure adapted to serve as a fusion plate for tissue re-growth into said micro-lattice hollow structure.

3. A vertebral fixation apparatus according to Claim 1, wherein said fixation plate is formed with a plurality of fixing holes, each said fixing hole having a screw inserted therethrough for fixing said fixation plate on a vertebral portion.

4. A vertebral fixation apparatus according to Claim 3, wherein each said fixing hole is obliquely formed through said fixation plate so that each said screw is obliquely locked into said vertebral portion through said fixing hole as obliquely formed.

5. A vertebral fixation apparatus according to Claim 3, wherein each said fixing hole is jacketed therein with a screw sleeve so that each said screw is locked into said vertebral portion by passing said screw through said screw sleeve.

6. A vertebral fixation apparatus according to Claim 3, wherein said fixing hole is formed with female threads therein to be engaged with male threads as formed on said screw.

7. A vertebral fixation apparatus according to Claim 2, wherein said fixation plate includes a solid metallic frame or wall fencing said micro-lattice hollow structure.

8. A vertebral fixation apparatus according to Claim 1, wherein each said saddle portion is protected with a packing or protection member along a periphery of said saddle portion for holding each said spinous process.

9. A vertebral fixation apparatus according to Claim 1, wherein said fixation plate is divided into two half plate members, which are adjustably combined and locked by a fastening screw by passing through a pair of screw openings formed through a pair of projections respectively formed on said two plate members.

10. A vertebral fixation apparatus according to Claim 9, wherein said two plate members include a superior plate member and an inferior plate member, both said plate members combinably fastened for holding adjacent spinous processes.

11. A vertebral fixation apparatus according to Claim 1, wherein said fixation plate is formed as a single-leaf plate member, which is combined with a holding means including a superior saddle portion and an inferior saddle portion adapted for holding adjacent spinous processes in said holding means.

12. A vertebral fixation apparatus according to Claim 1, wherein said fixation plate is formed with a recess in a bottom of said fixation plate to form a space for accommodating a spinal nerve spread under said fixation plate.

13. A vertebral fixation apparatus according to Claim 9, wherein a spring or a spacer is retained between said two projections.

14. A vertebral fixation apparatus according to Claim 1, wherein said fixation plate includes at least an inferior fixing hole adjacent a root portion of the inferior spinous process and obliquely formed in said fixation plate along a first axis which is generally projectively aligned with or parallel to an orientation of the inferior transverse process ; and a superior fixing hole adjacent to a root portion of the superior spinous process and obliquely formed in said fixation plate along a second axis which is generally projectively perpendicular to the first axis.
